# EUROPEAN PATENT APPLICATION

(11) **EP 4 354 457 A1**
(43) Date of publication of application: **17.04.2024**
(21) Application number: 23202327.5
(22) Date of filing: 09.10.2023
(51) Int. Cl.: G16H 50/30, G16H 20/70, G16H 40/63, G16H 50/20, G16H 50/70, G16H 40/67

(54) **SYSTEM AND METHOD TO DETECT AUTOMOTIVE STRESS AND/OR ANXIETY IN VEHICLE OPERATORS AND IMPLEMENT REMEDIATION MEASURES VIA THE CABIN ENVIRONMENT**

(30) Priority: 11.10.2022 US 202263379106 P
(71) Applicant: Harman International Industries, Incorporated, Stamford, CT 06901 (US)
(72) Inventor: MOLINSKA, Marta, 60-856 Poznan (PL); JULIANO, Julia, Troy, 48085 (US); HARIHARAKRISHNAN, Anilkumar, 82054 Sauerlach (DE)
(74) Representative: Rummler, Felix

(57) **Abstract**

Methods and systems of relieving stress for operators of vehicles are disclosed. In some embodiments, a set of inputs may be received from sensors after an operator begins operating a vehicle. The set of inputs may be processed to determine a stress level of the operator after vehicle operation begins. The stress level may be compared to a pre-determined stress level threshold. If the stress level is outside of the threshold and lower than the threshold, a first remediation measure may be implemented, and if the stress level is outside of the threshold and higher than the threshold, a different, second remediation measure may be implemented. Methods and systems of relieving stress for operators of vehicles are disclosed. A first set of sensor inputs may be received before an operator begins vehicle operation, and a second set of sensor inputs may be received after the operator begins vehicle operation. The first set of inputs may be processed to establish a baseline cognitive state, and the second set of inputs maybe processed to establish a current cognitive state. If the second set of inputs correlates to one or more physiological indicators of stress, and if a remediation measure will reduce a stress level of the operator based at least upon on a comparison of the current cognitive state to the baseline cognitive state, the remediation measure may be implemented.

## Description

### BACKGROUND and SUMMARY

Current vehicle cabins may not consider the physical and mental state of a vehicle operator. In particular, the stress and/or anxiety experienced by the vehicle operator while the vehicle is in operation can affect driver performance. Physiological parameters regarding the vehicle operator may be utilized by the vehicle to address or respond to the current physical and mental state of the vehicle operator. Adjustments to the vehicle cabin environment via an infotainment system and the like may reduce the effect of stressors on the physical and mental state of the vehicle operator while the vehicle is in operation.

As disclosed herein, a system and method for determining the stress level of the vehicle operator and/or implementing remediation measures responsive to one or more threshold levels of a driver's physical and/or mental state. As described, physiological parameters regarding the vehicle operator may be utilized to assess the stress level of the vehicle operator. For example, the physiological parameters may include heart rate, heart rate variability, breathing pattern, blood pressure, blood oxygenation, blood glucose levels, size of pupil diameter, muscle activity, forehand temperature, nose area temperature, brain activity, including alpha, beta, gamma, and theta waves, and the like.

The physiological parameters may be available in at least one of a continuous manner or a discontinuous manner. In particular, the physiological parameters may be available at specific phases experienced by the vehicle operators in the cabin environment, and/or before or after in the cabin environment. In an example, the system and method may define a baseline stress level experienced by the vehicle operator. More specifically, the initial state of the vehicle operator with regards to physical and mental wellbeing prior to entering the vehicle cabin may be utilized as the baseline stress level. Additionally, the baseline stress level may be developed based on information derived from questionnaires, health records, tasks, daily routines, and the like. The resultant stress level after entering the vehicle cabin and operating the vehicle may be based on changes characterized by either a combination of multiple physiological parameters or a combination of all the physiological parameters. The physiological parameters may be detected by visible or non-visible displays of stress level or anxiety of the vehicle operator.

After determining the current stress level of the vehicle operator, remediation measures may be implemented to alleviate stressors that may be responsible for the current stress level of the vehicle operator, where such implementations may also be based on the stress or anxiety level before entering the vehicle cabin. The remediation measures may be implemented via an infotainment system wherein the infotainment system may utilize pre-emptive information of the infotainment system to make adaptive changes to navigation, HMFdisplay content, vehicle temperature, airflow, scent, seat features, and the like.

It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The disclosure may be better understood from reading the following description of non-limiting embodiments, with reference to the attached drawings, wherein below:
FIG. 1 shows an example partial view of a vehicle cabin in accordance with one or more embodiments of the present disclosure;
FIG. 2 shows an example in vehicle computing system in accordance with one or more embodiments of the present disclosure; and
FIG. 3 shows a flow chart of a method of determining a cognitive state of a vehicle operator based on input received from a plurality of monitoring devices and implementing remediation measures, in accordance with one or more embodiments of the present disclosure

### DETAILED DESCRIPTION

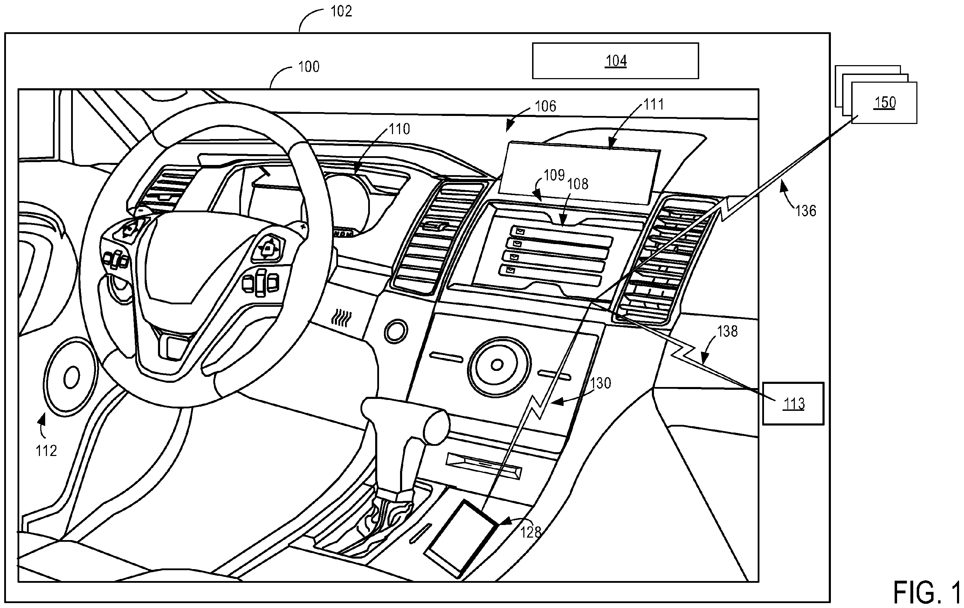

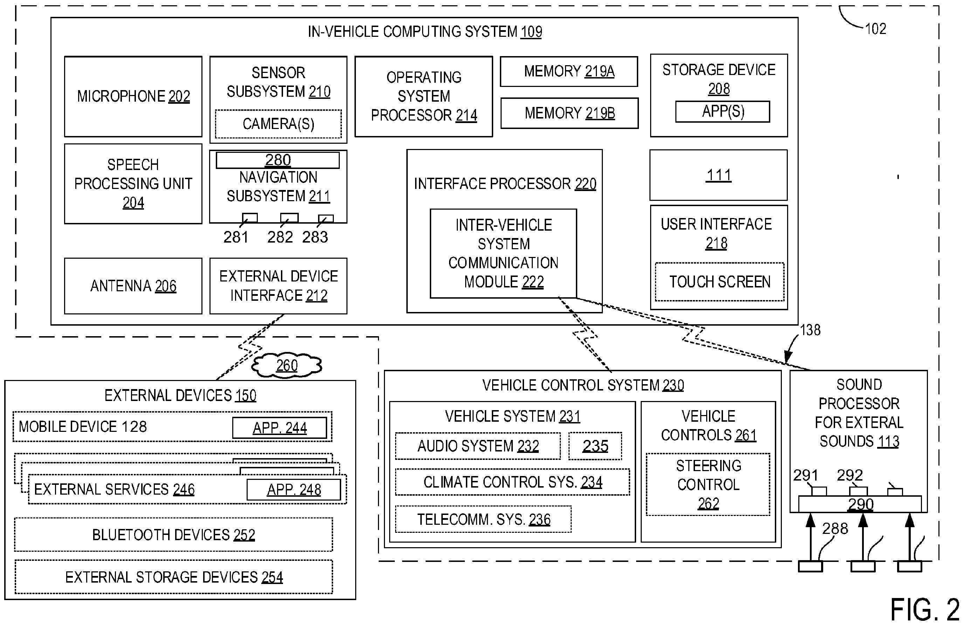

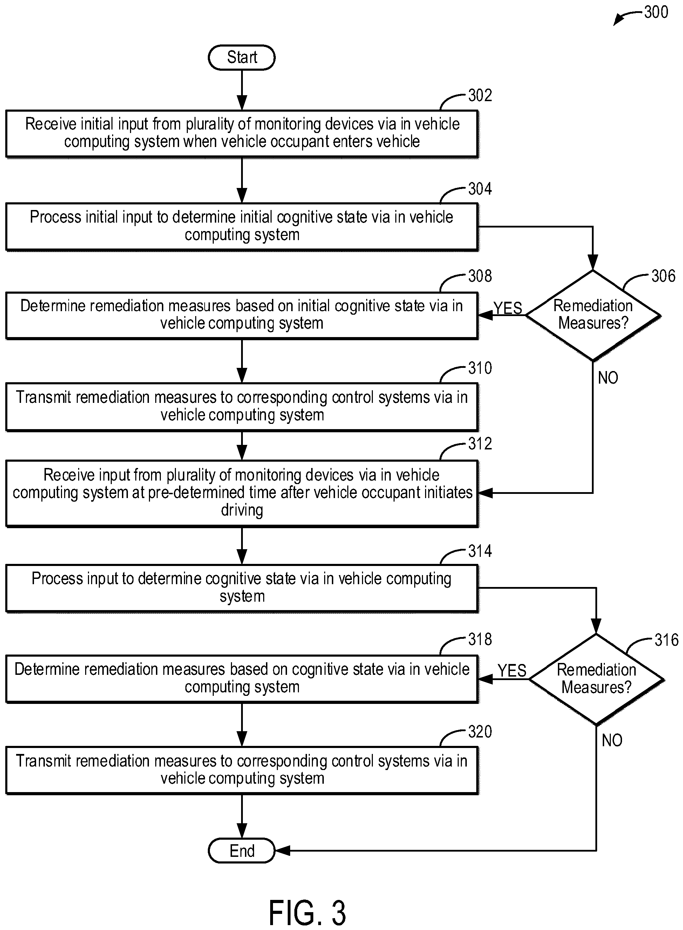

Disclosed herein are various systems and methods for determining a cognitive state of a vehicle operator and implementing remediation measures in response to detecting physiological indicators of stress. An example of a vehicle cabin wherein the vehicle operator may experience external stressors and internal stressors is illustrated in FIG. 1. An in vehicle computing system that may implement remediation measures in response to detecting physiological indicators of stress is illustrated in FIG. 2. As shown in FIG. 3, a method for determining physiological indicators of stress and implementing remediation measures in response to reduce stress level of the vehicle operator.

FIG. 1 shows an example partial view of one type of environment for an audio customization system: an interior of a cabin 100 of a vehicle 102, in which a driver and/or one or more passengers may be seated. Vehicle 102 of FIG. 1 may be a motor vehicle including drive wheels (not shown) and an internal combustion engine 104. Internal combustion engine 104 may include one or more combustion chambers which may receive intake air via an intake passage and exhaust combustion gases via an exhaust passage. Vehicle 102 may be a road automobile, among other types of vehicles. In some examples, vehicle 102 may include a hybrid propulsion system including an energy conversion device operable to absorb energy from vehicle motion and/or the engine and convert the absorbed energy to an energy form suitable for storage by an energy storage device. Vehicle 102 may include a fully electric vehicle, incorporating fuel cells, solar energy capturing elements, and/or other energy storage systems for powering the vehicle.

As shown, an instrument panel 106 may include various displays and controls accessible to a human driver (also referred to as the vehicle operator) of vehicle 102. For example, instrument panel 106 may include a touch screen 108 of an in vehicle computing system 109 (e.g., an infotainment system), an audio system control panel, and an instrument cluster 110. Touch screen 108 may receive user input to the in vehicle computing system 109 for controlling audio output, visual display output, vehicle operator preferences, control parameter selection, etc. While the example system shown in FIG. 1 includes audio system controls that may be performed via a user interface of in vehicle computing system 109, such as touch screen 108 without a separate audio system control panel, in other embodiments, the vehicle may include an audio system control panel, which may include controls for a conventional vehicle audio system such as a radio, compact disc player, MP3 player, etc. The audio system controls may include features for controlling one or more aspects of audio output via speakers 112 of a vehicle speaker system. For example, the in vehicle computing system or the audio system controls may control a volume of audio output, a distribution of sound among the individual speakers of the vehicle speaker system, an equalization of audio signals, and/or any other aspect of the audio output. In further examples, in vehicle computing system 109 may adjust a radio station selection, a playlist selection, a source of audio input (e.g., from radio or CD or MP3), etc., based on vehicle operator input received directly via touch screen 108, or based on data regarding the vehicle operator (such as a physical state and/or environment of the vehicle operator) received via external devices 150 and/or mobile device 128.

In addition, the in vehicle computing system 109 may adjust audio output volume or power output level, which speakers are activated, and signals for generating sounds at speakers in response to output from sound processor for external sounds 113. The audio system of the vehicle may include an amplifier (not shown) coupled to a plurality of loudspeakers (not shown). Sound processor for external sounds 113 may be connected to the in vehicle computing system via communication link 138 which may be wired or wireless, as discussed with reference to communication link 130, and configured to provide two-way communication between the sound processor for external sounds 113 and the in vehicle computing system 109.

In some embodiments, one or more hardware elements of in vehicle computing system 109, such as touch screen 108, a display screen 111, various control dials, knobs and buttons, memory, processor(s), and any interface elements (e.g., connectors or ports) may form an integrated head unit that is installed in instrument panel 106 of the vehicle. The head unit may be fixedly or removably attached in instrument panel 106. In additional or alternative embodiments, one or more hardware elements of the in vehicle computing system 109 may be modular and may be installed in multiple locations of the vehicle.

The cabin 100 may include one or more sensors for monitoring the vehicle, the vehicle operator, and/or the environment. For example, the cabin 100 may include one or more seat-mounted pressure sensors configured to measure the pressure applied to the seat to determine the presence of a vehicle operator, door sensors configured to monitor door activity, humidity sensors to measure the humidity content of the cabin, microphones to receive user input in the form of voice commands, to enable a vehicle operator to conduct telephone calls, and/or to measure ambient noise in the cabin 100, etc. It is to be understood that the above-described sensors and/or one or more additional or alternative sensors may be positioned in any suitable location of the vehicle. For example, sensors may be positioned in an engine compartment, on an external surface of the vehicle, and/or in other suitable locations for providing information regarding the operation of the vehicle, ambient conditions of the vehicle, a vehicle operator of the vehicle, etc. Information regarding ambient conditions of the vehicle, vehicle status, or vehicle driver may also be received from sensors external to/separate from the vehicle (that is, not part of the vehicle system), such as sensors coupled to external devices 150 and/or mobile device 128.

Cabin 100 may also include one or more user objects, such as mobile device 128, that are stored in the vehicle before, during, and/or after travelling. The mobile device 128 may include a smart phone, a tablet, a laptop computer, a portable media player, and/or any suitable mobile computing device. The mobile device 128 may be connected to the in vehicle computing system 109 via communication link 130. The communication link 130 may be wired (e.g., via Universal Serial Bus [USB], Mobile High-Definition Link [MHL], High-Definition Multimedia Interface [HDMI], Ethernet, etc.) or wireless (e.g., via BLUETOOTH, WIFI, WIFI direct, Near-Field Communication [NFC], cellular connectivity, etc.) and configured to provide two-way communication between the mobile device and the in vehicle computing system. The mobile device 128 may include one or more wireless communication interfaces for connecting to one or more communication links (e.g., one or more of the example communication links described above). The wireless communication interface may include one or more physical devices, such as antenna(s) or port(s) coupled to data lines for carrying transmitted or received data, as well as one or more modules/drivers for operating the physical devices in accordance with other devices in the mobile device. For example, the communication link 130 may provide sensor and/or control signals from various vehicle systems (such as vehicle audio system, climate control system, etc.) and the touch screen 108 to the mobile device 128 and may provide control and/or display signals from the mobile device 128 to the in vehicle systems and the touch screen 108. The communication link 130 may also provide power to the mobile device 128 from an in vehicle power source in order to charge an internal battery of the mobile device.

In vehicle computing system 109 may also be communicatively coupled to additional devices operated and/or accessed by the vehicle operator but located external to vehicle 102, such as one or more external devices 150. In the depicted embodiment, external devices are located outside of vehicle 102 though it will be appreciated that in alternate embodiments, external devices may be located inside cabin 100. The external devices may include a server computing system, personal computing system, portable electronic device, electronic wrist band, electronic head band, portable music player, electronic activity tracking device, pedometer, smart-watch, GPS system, etc. Further, the one or more external devices 150 may include a plurality of monitoring devices to identify and detect physiological indicators of stress of the vehicle operator.

External devices 150 may be connected to the in vehicle computing system via communication link 136 which may be wired or wireless, as discussed with reference to communication link 130, and configured to provide two-way communication between the external devices and the in vehicle computing system. For example, external devices 150 may include one or more sensors and communication link 136 may transmit sensor output from external devices 150 to in vehicle computing system 109 and touch screen 108. External devices 150 may also store and/or receive information regarding contextual data, physiological parameters of the vehicle operator, user behavior/preferences, operating rules, etc. and may transmit such information from the external devices 150 to in vehicle computing system 109 and touch screen 108.

In vehicle computing system 109 may analyze the input received from external devices 150, mobile device 128, sound processor for external sounds 113, and/or other input sources and select settings for various in vehicle systems (such as climate control system or audio system), provide output via touch screen 108 and/or speakers 112, communicate with mobile device 128 and/or external devices 150, and/or perform other actions based on the assessment. In some embodiments, all or a portion of the assessment may be performed by the mobile device 128 and/or the external devices 150.

In some embodiments, one or more of the external devices 150 may be communicatively coupled to in vehicle computing system 109 indirectly, via mobile device 128 and/or another of the external devices 150. For example, communication link 136 may communicatively couple external devices 150 to mobile device 128 such that output from external devices 150 is relayed to mobile device 128. Data received from external devices 150 may then be aggregated at mobile device 128 with data collected by mobile device 128. The aggregated data may then be transmitted to the in vehicle computing system 109 and touch screen 108 via communication link 130. Similar data aggregation may occur at a server system and then transmitted to in vehicle computing system 109 and touch screen 108 via communication link 136/130.

FIG. 2 shows a block diagram of an in vehicle computing system 109 configured and/or integrated inside vehicle 102. In vehicle computing system 109 may perform one or more of the methods described herein in some embodiments. In some examples, the in vehicle computing system 109 may be an infotainment system configured to provide information-based media content (audio and/or visual media content, including entertainment content, navigational services, etc.) to a vehicle operator to enhance the operator's in vehicle experience. The vehicle infotainment system may include, or be coupled to, various vehicle systems, sub-systems, hardware components, as well as software applications and systems that are integrated in, or integrated into, vehicle 102 in order to enhance an in vehicle experience for a driver and/or a passenger.

In vehicle computing system 109 may include one or more processors including an operating system processor 214 and an interface processor 220. Operating system processor 214 may execute an operating system on the in vehicle computing system, and control input/output, display, playback, and other operations of the in vehicle computing system. Interface processor 220 may interface with a vehicle control system 230 and sound processor for external sounds 113 via an inter-vehicle system communication module 222.

Inter-vehicle system communication module 222 may output data to other vehicle systems 231 and vehicle control elements 261, while also receiving data input from other vehicle components and systems 231, 261, e.g. by way of vehicle control system 230. When outputting data, inter-vehicle system communication module 222 may provide a signal via a bus corresponding to any status of the vehicle, the vehicle surroundings, or the output of any other information source connected to the vehicle. Vehicle data outputs may include, for example, analog signals (such as current velocity), digital signals provided by individual information sources (such as clocks, thermometers, location sensors such as Global Positioning System [GPS] sensors, etc.), digital signals propagated through vehicle data networks (such as an engine CAN bus through which engine related information may be communicated, a climate control CAN bus through which climate control related information may be communicated, and a multimedia data network through which multimedia data is communicated between multimedia components in the vehicle). For example, the in vehicle computing system 109 may retrieve from the engine CAN bus the current speed of the vehicle estimated by the wheel sensors, a power state of the vehicle via a battery and/or power distribution system of the vehicle, an ignition state of the vehicle, etc. In addition, other interfacing means such as Ethernet may be used as well without departing from the scope of this disclosure.

A non-volatile storage device 208 may be included in in vehicle computing system 109 to store data such as instructions executable by processors 214 and 220 in non-volatile form. The storage device 208 may store application data, including prerecorded sounds, to enable the in vehicle computing system 109 to run an application for connecting to a cloud-based server and/or collecting information for transmission to the cloud-based server. The application may retrieve information gathered by vehicle systems/sensors, input devices (e.g., user interface 218), data stored in volatile 219A or non-volatile storage device (e.g., memory) 219B, devices in communication with the in vehicle computing system (e.g., a mobile device connected via a Bluetooth link), etc. In vehicle computing system 109 may further include a volatile memory 219A. Volatile memory 219A may be random access memory (RAM). Non-transitory storage devices, such as non-volatile storage device 208 and/or non-volatile memory 219B, may store instructions and/or code that, when executed by a processor (e.g., operating system processor 214 and/or interface processor 220), causes the in vehicle computing system 109 to perform one or more of the actions described in the disclosure.

A microphone 202 may be included in the in vehicle computing system 109 to receive voice commands from a vehicle operator, to measure ambient noise in the vehicle, to determine whether audio from speakers of the vehicle is tuned in accordance with an acoustic environment of the vehicle, etc. A speech processing unit 204 may process voice commands, such as the voice commands received from the microphone 202. In some embodiments, in vehicle computing system 109 may also be able to receive voice commands and sample ambient vehicle noise using a microphone included in an audio system 232 of the vehicle.

One or more additional sensors may be included in a sensor subsystem 210 of the in vehicle computing system 109. For example, the sensor subsystem 210 may include a camera, such as a rear view camera for assisting a vehicle operator in parking the vehicle and/or a cabin camera for identifying a vehicle operator (e.g., using facial recognition and/or vehicle operator gestures). Sensor subsystem 210 of in vehicle computing system 109 may communicate with and receive inputs from various vehicle sensors and may further receive user inputs. For example, the inputs received by sensor subsystem 210 may include transmission gear position, transmission clutch position, gas pedal input, brake input, transmission selector position, vehicle speed, engine speed, mass airflow through the engine, ambient temperature, intake air temperature, etc., as well as inputs from climate control system sensors (such as heat transfer fluid temperature, antifreeze temperature, fan speed, passenger compartment temperature, desired passenger compartment temperature, ambient humidity, etc.), an audio sensor detecting voice commands issued by a vehicle operator, a fob sensor receiving commands from and optionally tracking the geographic location/proximity of a fob of the vehicle, etc. While certain vehicle system sensors may communicate with sensor subsystem 210 alone, other sensors may communicate with both sensor subsystem 210 and vehicle control system 230, or may communicate with sensor subsystem 210 indirectly via vehicle control system 230.

A navigation subsystem 211 of in vehicle computing system 109 may generate and/or receive navigation information such as location information (e.g., via a GPS sensor and/or other sensors from sensor subsystem 210), route guidance, traffic information, point-of-interest (POI) identification, and/or provide other navigational services for the driver. Navigation sub-system 211 may include inputs/outputs 280, including analog to digital converters, digital inputs, digital outputs, network outputs, radio frequency transmitting devices, etc. The sound processor for external sounds 113 may also include a central processing unit 281, volatile memory 282, and non-volatile (e.g., non-transient memory) 283.

External device interface 212 of in vehicle computing system 109 may be coupled to and/or communicate with one or more external devices 150 located external to vehicle 102. While the external devices are illustrated as being located external to vehicle 102, it is to be understood that they may be temporarily housed in vehicle 102, such as when the vehicle operator is operating the external devices while operating vehicle 102. In other words, the external devices 150 are not integral to vehicle 102. As one example, the external devices 150 may include a plurality of monitoring devices that identify and detect physiological indicators of stress of the vehicle operator. The external devices 150 may include a mobile device 128 (e.g., connected via a Bluetooth, NFC, WIFI direct, or other wireless connection) or an alternate Bluetooth-enabled device 252. Mobile device 128 may be a mobile phone, smart phone, wearable devices/sensors that may communicate with the in vehicle computing system via wired and/or wireless communication, or other portable electronic device(s). Other external devices include external services 246. For example, the external devices may include extravehicular devices that are separate from and located externally to the vehicle. Still other external devices include external storage devices 254, such as solid-state drives, pen drives, USB drives, etc. External devices 150 may communicate with in vehicle computing system 109 either wirelessly or via connectors without departing from the scope of this disclosure. For example, external devices 150 may communicate with in vehicle computing system 109 through the external device interface 212 over network 260, a universal serial bus (USB) connection, a direct wired connection, a direct wireless connection, and/or other communication link.

The external device interface 212 may provide a communication interface to enable the in vehicle computing system 109 to communicate with mobile devices associated with contacts of the driver. For example, the external device interface 212 may enable phone calls to be established and/or text messages (e.g., SMS, MMS, etc.) to be sent (e.g., via a cellular communications network) to a mobile device associated with a contact of the driver. The external device interface 212 may additionally or alternatively provide a wireless communication interface to enable the in vehicle computing system to synchronize data with one or more devices in the vehicle (e.g., the driver's mobile device) via WIFI direct.

One or more applications 244 may be operable on mobile device 128. As an example, mobile device application 244 may be operated to aggregate user data regarding interactions of the vehicle operator with the mobile device. For example, mobile device application 244 may aggregate data regarding music playlists listened to by the vehicle operator on the mobile device, telephone call logs (including a frequency and duration of telephone calls accepted by the vehicle operator), positional information including locations frequented by the vehicle operator and an amount of time spent at each location, etc. The collected data may be transferred by application 244 to external device interface 212 over network 260. In addition, specific user data requests may be received at mobile device 128 from in vehicle computing system 109 via the external device interface 212. The specific data requests may include requests for determining where the vehicle operator is geographically located, an ambient noise level and/or music genre at the vehicle operator's location, an ambient weather condition (temperature, humidity, etc.) at the vehicle operator's location, etc. Mobile device application 244 may send control instructions to components (e.g., microphone, amplifier etc.) or other applications (e.g., navigational applications) of mobile device 128 to enable the requested data to be collected on the mobile device or requested adjustment made to the components. Mobile device application 244 may then relay the collected information back to in vehicle computing system 109.

Likewise, one or more applications 248 may be operable on external services 246. As an example, external services applications 248 may be operated to aggregate and/or analyze data from multiple data sources. For example, external services applications 248 may aggregate data from one or more social media accounts of the vehicle operator, data from the in vehicle computing system (e.g., sensor data, log files, user input, etc.), data from an internet query (e.g., weather data, POI data), etc. The collected data may be transmitted to another device and/or analyzed by the application to determine a context of the driver, vehicle, and environment and perform an action based on the context (e.g., requesting/sending data to other devices).

Vehicle control system 230 may include controls for controlling aspects of various vehicle systems 231 involved in different in vehicle functions. These may include, for example, controlling aspects of vehicle audio system 232 for providing audio entertainment to the vehicle operators, aspects of climate control system 234 for meeting the cabin cooling or heating needs of the vehicle operators, as well as aspects of telecommunication system 236 for enabling vehicle operators to establish telecommunication linkage with others.

Audio system 232 may include one or more acoustic reproduction devices including electromagnetic transducers such as speakers 235. Vehicle audio system 232 may be passive or active such as by including a power amplifier. In some examples, in vehicle computing system 109 may be the only audio source for the acoustic reproduction device or there may be other audio sources that are connected to the audio reproduction system (e.g., external devices such as a mobile phone). The connection of any such external devices to the audio reproduction device may be analog, digital, or any combination of analog and digital technologies.

Climate control system 234 may be configured to provide a comfortable environment within the cabin or passenger compartment of vehicle 102. Climate control system 234 includes components enabling controlled ventilation such as air vents, a heater, an air conditioner, an integrated heater and air-conditioner system, etc. Other components linked to the heating and air-conditioning setup may include a windshield defrosting and defogging system capable of clearing the windshield and a ventilation-air filter for cleaning outside air that enters the passenger compartment through a fresh-air inlet.

Vehicle control system 230 may also include controls for adjusting the settings of various vehicle controls 261 (or vehicle system control elements) related to the engine and/or auxiliary elements within a cabin of the vehicle, such as steering wheel controls 262 (e.g., steering wheel-mounted audio system controls, cruise controls, windshield wiper controls, headlight controls, turn signal controls, etc.), instrument panel controls, microphone(s), accelerator/brake/clutch pedals, a gear shift, door/window controls positioned in a driver or passenger door, seat controls, cabin light controls, audio system controls, cabin temperature controls, and the like. Vehicle controls 261 may also include internal engine and vehicle operation controls (e.g., engine controller module, actuators, valves, etc.) that are configured to receive instructions via the CAN bus of the vehicle to change operation of one or more of the engine, exhaust system, transmission, and/or other vehicle system. The control signals may also control audio output at one or more speakers 235 of the vehicle's audio system 232. For example, the control signals may adjust audio output characteristics such as volume, equalization, audio image (e.g., the configuration of the audio signals to produce audio output that appears to a vehicle operator to originate from one or more defined locations), audio distribution among a plurality of speakers, etc. Likewise, the control signals may control vents, air conditioner, and/or heater of climate control system 234. For example, the control signals may increase delivery of cooled air to a specific section of the cabin.

Control elements positioned on an outside of a vehicle (e.g., controls for a security system) may also be connected to computing system 109, such as via communication module 222. The control elements of the vehicle control system may be physically and permanently positioned on and/or in the vehicle for receiving user input. In addition to receiving control instructions from in vehicle computing system 109, vehicle control system 230 may also receive input from one or more external devices 150 operated by the vehicle operator, such as from mobile device 128. This allows aspects of vehicle systems 231 and vehicle controls 261 to be controlled based on vehicle operator input received from the external devices 150.

In vehicle computing system 109 may further include an antenna 206. Antenna 206 is shown as a single antenna, but may comprise one or more antennas in some embodiments. The in vehicle computing system may obtain broadband wireless internet access via antenna 206, and may further receive broadcast signals such as radio, television, weather, traffic, and the like. The in vehicle computing system may receive positioning signals such as GPS signals via one or more antennas 206. The in vehicle computing system may also receive wireless commands via FR such as via antenna(s) 206 or via infrared or other means through appropriate receiving devices. In some embodiments, antenna 206 may be included as part of audio system 232 or telecommunication system 236. Additionally, antenna 206 may provide AM/FM radio signals to external devices 150 (such as to mobile device 128) via external device interface 212.

One or more elements of the in vehicle computing system 109 may be controlled by a vehicle operator via user interface 218. User interface 218 may include a graphical user interface presented on a touch screen, such as touch screen 108 of FIG. 1, and/or user-actuated buttons, switches, knobs, dials, sliders, etc. For example, user-actuated elements may include steering wheel controls, door and/or window controls, instrument panel controls, audio system settings, climate control system settings, and the like. A vehicle operator may also interact with one or more applications of the in vehicle computing system 109 and mobile device 128 via user interface 218. In addition to receiving a vehicle operator's vehicle setting preferences on user interface 218, vehicle settings selected by in vehicle control system may be displayed to a vehicle operator on user interface 218. Notifications and other messages (e.g., received messages), as well as navigational assistance, may be displayed to the vehicle operator on a display of the user interface. Vehicle operator preferences/information and/or responses to presented messages may be performed via user input to the user interface.

Sound processor for external sounds 113 may be electrically coupled to a plurality of microphones 288 that are external to vehicle 102 (e.g., external microphones). Sound processor for external sounds 113 may receive signals output from each of external microphones 288 and convert the signals into an angle value, a distance value, and a type identifier for sound sources that are external to vehicle 102. Sound processor for external sounds 113 may output angle data, distance data, and sound source type data to in vehicle computing system 109 via communication link 138. However, in other examples, the tasks and functions that may be performed by sound processor for external sounds 113 may be integrated into in vehicle computing system 109. In addition, external microphones 288 may be in direct electric communication with in vehicle computing system 109 in such examples.

Sound processor for external sounds 113 may include inputs/outputs 290, including analog to digital converters, digital inputs, digital outputs, network outputs, radio frequency transmitting devices, etc. The sound processor for external sounds 113 may also include a central processing unit 291, volatile memory 292, and non-volatile 293 (e.g., non-transient memory).

FIG. 3 is a flow chart of a method 300 of operating an in vehicle computing system to control one or more vehicle systems based on input from a plurality of monitoring devices and/or pre-determined information. The plurality of monitoring devices may include various senses configured to obtain physiological parameters regarding the vehicle operator. In other embodiment, the method 300 may be applied to vehicle occupants other than the vehicle operator. The physiological parameters may be pre-determined via questionnaires, health records, tasks, daily routines, and the like. For example, method 300 may be performed by in vehicle computing system 109 of FIG. 2 based on input from a monitoring devices.

At 302, the method 300 includes receiving initial input from a plurality of monitoring devices when a vehicle operator enters the vehicle. Specifically, the initial input may be received at the in vehicle computing system from one or more the monitoring devices. The monitoring devices may be worn by a vehicle operator and may include one or more sensors such as a heart rate sensor, a forehand temperature sensor, a blood pressure sensor, a blood glucose sensor, a nose area temperature sensor, a blood oxygenation sensor, a brain wave sensor, a perspiration level sensor, and the like. Other examples of monitoring device may include a camera that monitors muscular activity and pupil activity or pupil diameter of the vehicle operator.

The initial input may not include sensor signals corresponding to all of the various physiological parameters discussed above. The initial input may include sensor signals corresponding to a subset of the physiological parameters discussed above. For example, only heart rate, perspiration level, and temperature before the vehicle operating begins operating the vehicle may be considered initially. However, after the vehicle operator beings operating the vehicle, the input received from the plurality of monitoring devices may include sensor signals corresponding to additional or alternative physiological parameters, such as blood oxygenation, muscle activity, and the like.

Receiving initial input from the monitoring devices includes receiving sensor signals from the various wearable sensors based on interaction of the vehicle operator with the monitoring devices. In one example wherein the monitoring devices is communicatively coupled to the in vehicle computing system via a communication link or network, receiving initial input from the monitoring devices may include directly receiving initial input from the monitoring devices. In another example wherein the monitoring devices is communicatively coupled to a mobile device (e.g., via a communication link or network) and the mobile device, in turn, is communicatively coupled to the in vehicle computing system via a communication link or network, receiving initial input from the monitoring devices includes indirectly receiving initial input from the monitoring devices via the mobile device.

At 304, the method includes processing the initial input to determine cognitive state via the in vehicle computing system. The initial input received from the monitoring devices may be indicative of various aspects of the physical state of the vehicle operator including, but not limited to heart rate, heart rate variability, breathing pattern, blood pressure, blood oxygenation, blood glucose levels, size of pupil diameter, muscle activity, forehand temperature, nose area temperature, and brain activity, including alpha, beta, gamma, and theta waves. Different changes to the various aspects of the physical state of the vehicle operator described above may allow the cognitive state of the vehicle operator to be determined. In some embodiments, the combination of different changes to the various aspects of the physical state may have a pattern correlated to stress due to operating a vehicle. Stress due to operating a vehicle may be referred to herein as vehicle stress. Vehicle stress may be related to road conditions, traffic, road construction and detours, and the like.

A stress level of the vehicle operator may be determined in response to changes in the various aspects of the physical state. The initial input may be processed to establish a baseline of the cognitive state of the vehicle operator prior to the vehicle operator entering the vehicle. Specifically, the initial input may be processed to determine the stress level of the vehicle operator prior to entering the vehicle. In particular, the in vehicle computing system may determine whether the initial input values correlate to physiological indicators of stress. For example, increased heat rate, reduced high frequent components of heart rate variability, increased blood pressure, suppression of alpha waves and increasing theta waves of the brain, increase in forehand temperature, decrease in nose area temperature, and increase in difference between the forehand temperature and nose area temperature may indicate a higher stress level of the vehicle operator. Similarly, increased muscular activity, increased pupil diameter, decreased standard deviation of pupil diameter, increased blinking, later gaze disengagement, and increased peak saccade velocity may indicate a higher stress level of the vehicle operator.

In some embodiments of the present disclosure, the initial input and input at different points during the operation of the vehicle may be utilized to identify and detect the physiological indicators of stress. Further, the physiological indicators of stress may be utilized to establish a pre-determined stress level threshold of the vehicle operator. In this way, a desirable stress level of the vehicle operator may be established and maintained during operation of the vehicle. For example, prior to entering the vehicle, the vehicle operator may be at a stress level lower than the pre-determined stress level threshold.

Driving at a stress lower than the pre-determined stress level threshold may decrease driving performance of the vehicle operator. In this case, the vehicle operator may not be aware of other vehicles or the surrounding area. Similarly, driving at a stress level higher than the pre-determined stress level threshold may decrease driving performance of the vehicle operator. In this case, the vehicle operator may be hyperaware of other vehicles and the surrounding area, which may negatively affect driving performance of vehicle operators of other vehicles. Driving at a stress level within the pre-determined stress level threshold may result in a desirable driving performance of the vehicle operator. It may be desirable to maintain the stress level of the vehicle operator with the pre-determined stress level threshold.

However, the pre-determined stress level threshold may be relative in that the pre-determined stress level threshold may be different in the vehicle compared to outside of the vehicle. In particular, the physiological parameters and physiological indicators that are indicative of vehicle stress may be different than the physiological parameters and physiological indicators of non-vehicle stress. Additionally, the stressors that results in undesirable stress levels may differ based on whether the vehicle operator is exposed to the stressor inside or outside of the vehicle. As such, a stressor outside the vehicle may not be a stressor inside the vehicle. Further, the remediation measures may affect the stress level of the vehicle operator differently depending on whether they are implemented inside the vehicle or outside of the vehicle. The same stimulus or action in the vehicle may result in a different response outside of the vehicle. For example, playing soothing audio outside of the vehicle may reduce the stress level of the vehicle operator until it is within the pre-determined stress level threshold. However, playing the same soothing audio inside of the vehicle may increase the stress level of the vehicle operator beyond the initial cognitive state and stress level. An in vehicle computing system (e.g., infotainment system) with adaptive learning capabilities may address the variability of the vehicle operator's physiological response to different stressors and remediation measures.

At 306, the method 300 includes determining whether remediation measures may be implemented. Determining whether remediation measures may be implemented may be based on whether the initial input values correlate to physiological indicators of stress and the corresponding stress level of the vehicle operator is within the pre-determined threshold. In some embodiments, determining whether remediation measures may reduce undesired stress levels may also be based on historical information, location data, and environmental factors, such as, the current and expected weather at the location, ambient temperature and humidity conditions, ambient noise level, etc. In some embodiments, all the physiological indicators of stress may be considered when determining whether remediation measures may be implemented.

In other embodiments, the physiological indicators may be grouped into a plurality of parameter sets wherein a subset of the physiological indicators are included in one parameter set. The initial input may include a subset of the physiological indicators of stress. In this way, parameter sets may be determined wherein the physiological indicators of stress are more closely related to vehicle stress instead of other external sources of stress or internal sources of stress that are unrelated to stress involved with operating a vehicle. Accordingly, the method 300 may utilize historical information, location data, medical records, and the like to identify particular stressors that individually affect a particular vehicle operator. Different stressors may affect different vehicle operators in different ways. To account for these differences, the parameter sets may be personalized and include a subset of the physiological indicators of stress that are more highly correlated with a higher stress level of the vehicle occupant. The subset of physiological indicators of stress included in the parameter sets may vary for different vehicle operators.

Further, the initial input values may correlate to physiological indicators of stress and being within the pre-determined stress level threshold without experiencing an undesirable stress level due to operating a vehicle. The vehicle operator may experience a plurality of external stressors or a plurality of internal stressors such as physical health, mental health (e.g., chronic anxiety), distractions (e.g., moving objects within the vehicle), work-related stress, other vehicle occupants, and the like. As one example, the vehicle operator may have an increased heart rate due to increased physical activity. As described above, the in vehicle computing system may be communicatively coupled to a mobile device. In this way, the mobile device may transmit location data to the in vehicle computing system indicating that the vehicle operator may have been at a fitness center, a gym, a park, and the like. Further, the mobile device may transmit fitness data. Additionally, one of the plurality of monitoring devices may indicate higher heart rate or higher perspiration levels. In this case, the physiological indicators of stress may return to a normal level without implementing remediation measures after a duration of time.

As another example, the vehicle operator may have an increased heart rate due to environmental stressors and the like. In particular, the vehicle operator may be entering the vehicle after experiencing a stressful environment (e.g., a hospital or work-related). Historical information and location data may identify locations correlating to higher stress levels of the vehicle operator and/or locations correlating to physiological indicators of stress that falsely simulate higher stress levels of the vehicle operator. Accordingly, the physiological indicators may return to a normal level without implementing remediation measures after a duration of time wherein the vehicle operator is not in contact with the stressor.

In contrast, the historical information and location data may not identify a location correlating to higher stress levels of the vehicle operator and/or locations correlating to physiological indicators of stress that falsely simulate higher stress levels of the vehicle operator. As such, the physiological indicators of stress may indicate higher stress levels of the vehicle operator that exceed the desired pre-determined stress level threshold. Additionally, the remediation measures may be implemented in response to the stress level of the vehicle occupant being below the pre-determined stress level threshold. In this way, historical information, location data, and the like may be utilized to adaptively learn the stressors of the particular vehicle operator that correlate to higher stress levels of the vehicle operator that exceed the pre-determined stress level threshold and lower stress levels of the vehicle operator that are below the pre-determined stress level threshold.

If it is determined physiological indicators are within the pre-determined stress level threshold, the method 300 includes receiving input from the plurality of monitoring devices via in-vehicle computing system at pre-determined time after the vehicle operator initiates driving at 312. If it is determined that physiological indicators of stress are not within the pre-determined stress level threshold, the method 300 includes determining remediation measures based on the initial cognitive state of the vehicle operator via in vehicle computing system at 308.

At 308, the method includes, determining remediation measures based on the initial cognitive state via the in vehicle computing system. To establish a normal cognitive state of the vehicle operator, a plurality of vehicle settings and functions may be adjusted to alleviate stressors. The vehicle settings may include, for example, a climate control system setting to adjust a cabin temperature, air flow, and the like, an audio system setting, a navigation system to adjust a drive route, and the like. Vehicle settings and functions of the infotainment system, including media volume and infotainment content, such as media playlists, entertainment, news, audio, video, and the like, of the infotainment system may be adjusted to establish the normal cognitive state of the vehicle operator.

The climate control system setting may be adjusted in response to selecting air conditioner, heater, and vent settings to adjust a cabin temperature so as to provide a selected level of cooling/heating and/or rate of cooling/heating. Accordingly, air conditioner settings may be selected to provide the determined temperature setting to a determined section of the cabin (e.g., entire cabin or driver area). In an alternate example, the in-vehicle control system may select a default "cool" setting in response to the elevated physical activity level of the vehicle operator, while overriding the climate control settings last selected by the vehicle operator (such as before exiting the vehicle).

The audio system settings may be adjusted in response to an elevated ambient noise level (e.g., higher than the threshold level), an elevated cabin noise level, and/or undesirable stress levels of the vehicle operator. In this way the audio source and audio system settings may be adjusted to provide a quieter or more soothing drive experience for the vehicle operator. For example, if the vehicle is in a noisy location or the noise level in the vehicle cabin is elevated, audio settings may be adjusted to mask the ambient noise or the noise level in the vehicle cabin to reduce the vehicle operator stress level and dissatisfaction. Additionally, the driving experience of the vehicle operator may be increased by automatically playing soothing and calm music, or a music genre or playlist that is marked as a vehicle operator favorite to alleviate stressors associated with elevated ambient noise level and cabin noise level.

Further, the navigation system may be adjusted in response to undesirable stress levels of the vehicle operator. In one embodiment, ameliorating stressors by adjusting a route based on weather and avoiding unplowed or flooded roads may reduce undesirable stress levels of the vehicle operator. In further embodiments, bringing attention back to driving may alleviate undesirable stress levels by providing additional routes that are faster and less stressful within driving range and/or based on previous destinations. In another embodiment, undesirable stress levels may be alleviated by predicting stressors. In particular, providing early warnings for emergency medical systems (EMS) activity, providing early lane change information, and predicting when weather might impact vision may alleviate stressor. In another embodiment, increasing travel expectations may alleviate undesirable stress levels may be alleviated by providing adaptive navigation instructions, providing early alerts to street closures and parking information near destination, providing estimations for driving distance based on fuel/ energy level and consumptions. In another embodiment, increasing road assistance may alleviate undesirable stress levels may be alleviated by making suggestions of fuel or charging stations nearby and/or along route based on fuel/energy level and consumption.

Further, the input received from the plurality of monitoring devices may be integrated with Advance Driver Assistance Systems (ADAS) wherein the ADAS may provide supportive, preventative, and/or curative interventions via adaptive, predictive, and pre-emptive changes of the ADAS settings to adjust the pre-determined stress threshold and establish the normal cognitive state of the vehicle operator. In particular, personalized intervention strategies may be developed wherein a driver model is developed based on driving behaviors, including braking patterns, acceleration patterns, steering patterns, and the like; the driver model is integrated with the information regarding the cognitive state of the vehicle operator and applicable standards to prevent degradation of driving performance to develop the personalization intervention strategies. The applicable standards to prevent degradation of driving performance may be defined by customization of actuator control patterns of a personalized envelope.

In particular, the ADAS of the vehicle may provide intervention to alleviate ameliorate stressors, bring attention back to driving, implement anxiety-reducing techniques, predict stressors, increase travel expectations, and provide increased road assistance similar to the in vehicle computing system. In particular, the ADAS may alleviate ameliorate stressors by starting autonomous features when visibility is low and automatically turning on controls for defogging or unfreezing windows. The ADAS may bring attention back to driving by providing visual and audio cures and suggesting an appropriate speed range (e.g., faster speed if too anxious, slower speed if angry, and average speed to avoid energy consumption). The anxiety reducing techniques may assist with sign translation (e.g., parking information) and adaptive ADAS sensitivity based on anxiety level. The ADAS may predict stressors by predicting pedestrian behavior and providing updates based on traffic information. Further, the increased travel expectation may assist with highway interchanges, merges, one-way streets, and maneuvering through traffic in addition to adaptive AR lines and lanes assist based on anxiety and contextual information. Assisting with parking and drawing attention to objects that may pose a threat may be implemented via increased road assistance.

The vehicle settings may be adjusted via voice personal assistants (VPA). VPAs may be an important component of an HMI in a vehicle cabin. Specifically, the input received from the plurality of monitoring devices may be integrated with VPA wherein the VPA may provide supportive, preventative, and/or curative interventions to adjust the pre-determined stress threshold and establish the normal cognitive state of the vehicle operator. The VPA may be integrated with the information regarding the cognitive state of the vehicle operator to realize applicable standards to prevent degradation of driving performance.

For example, the VPA of the vehicle may provide intervention to alleviate ameliorate stressors, bring attention back to driving, implement anxiety-reducing techniques, predict stressors, increase travel expectations, and provide increased road assistance similar to the in vehicle computing system and VPA. In particular, the VPA may alleviate ameliorate stressors by reappraising anger provoking events and playing shared music based on all passengers in car. The VPA may bring attention back to driving by providing a guided activity that keeps drivers focused on the road but distracted from work and providing feedback on both acceptable and unacceptable driving performance.

The anxiety reducing techniques may provide guided biofeedback exercises, venting sessions, positive psychology, and guided meditation in addition to assisting with planning, managing, and guiding through daily or work-related tasks and assisting with better posture and mood. The VPA may predict stressors based on a current time, weather, history of driver, calendar information, and sending alert messages to a mobile device. Further, the increased travel expectation may be based on history of lateness, sending messages to the mobile device to alert the vehicle operator when to leave, by syncing with the calendar and providing an option to notify the vehicle operator of appointments if running late, assisting passengers to increase productivity of passengers when acting as co-pilots, and providing features to pay for energy/fuel via a vehicle application. Providing information on additional time to destination and including an adaptive simplified gauge cluster screen and driving instructions may be implemented via increased road assistance.

Vehicle settings and configurations of the infotainment system may be implemented via adaptive, predictive, and/or pre-emptive changes. The input received from the plurality of monitoring devices may be integrated with infotainment system wherein the infotainment system may provide supportive, preventative, and/or curative interventions to adjust the pre-determined stress threshold and establish the normal cognitive state of the vehicle operator. The infotainment system may be integrated with the information regarding the cognitive state of the vehicle operator to realize applicable standards to prevent degradation of driving performance.

Specifically, the infotainment system of the vehicle may provide intervention to alleviate ameliorate stressors, bring attention back to driving, implement anxiety-reducing techniques, predict stressors, increase travel expectations, and provide increased road assistance similar to the in vehicle computing system, the ADAS, and VPA. In particular, the infotainment system may alleviate ameliorate stressors by automatically providing a prompt to reduce audio volume in high stress/anxiety situations. The infotainment system may bring attention back to driving by providing a prompt to change a podcast to music and/or turning distracting music off. The anxiety reducing techniques may provide options to noise cancel passengers, avoid calls on speaker, and change EQ settings to reduce sharp noises, and provide a prompt to turn on seat functions, such as massaging, heating, and cooling functionalities of the seats. The infotainment system may predict stressors by automatically adjusting temperature based on driver preference wherein the temperature may be adjusted by opening windows and temperature based on anxiety levels of the vehicle operator. Further, the increased travel expectation may pre-cool or pre-heat the vehicle based on the weather. Adjusting vehicle climate settings based on weather may be implemented via increased road assistance.

Additionally, the vehicle settings described above may be adjusted to increase the stress level of the vehicle operator that is below the pre-determined stress level threshold to maintain the stress level of the vehicle operator at the desired level. For example, the vehicle operator may not be at a desired awareness level to operate the vehicle optimally. As such, the climate control settings may automatically be adjusted to either increase or decrease the temperature of the vehicle cabin to increase awareness of the vehicle operator. Further, the audio system may play audio and increase the volume of audio that may be playing in the vehicle cabin to increase awareness of the vehicle operator.

At 310, the method includes transmitting remediation measures to corresponding systems via in vehicle computing system. The in vehicle computing system may implement the remediation measures to the appropriate vehicle systems to apply the selected settings to the target vehicle systems. For example, if the input from the monitoring devices is indicative of higher than normal forehand and nose area temperatures of the vehicle operator, the in vehicle computing system may adjust the temperature in the vehicle cabin. Thus, the in vehicle computing system may automatically adjust the cabin climate control settings to increase cabin cooling, or increase flow of cooled air to the vehicle operator area of the cabin. Accordingly, settings for the air vents, and an air conditioner may be determined and transmitted to the climate control system.

In this way, the in vehicle computing system automatically adjusts the vehicle settings by receiving sensed information from a plurality of monitoring devices and/or a mobile device and by using the sensed information before receiving some sort of user command from the vehicle operator. This includes before receiving a command from the vehicle operator at the in vehicle computing system, such as via the vehicle operator pushing a button, or touch screen, etc., as well as before receiving a command from the vehicle operator at the in vehicle computing system via the monitoring devices or the mobile device, such as via the vehicle operator pressing a button of the mobile device, providing a voice command from the mobile device to the in vehicle computing system, and the like.

While the method of FIG. 3 shows settings being determined based on vehicle operator input received from a monitoring devices, it will be appreciated that additional user input may also be received from a mobile device. For example, additional input regarding various aspects of the vehicle operator (such as the physical state of the vehicle operator and/or input indicative of an environment of the vehicle operator) may be received at the in vehicle computing system from the mobile device via a communication link or network. As an example, the input received from the mobile device may be indicative of environmental parameters of the vehicle operator such as an ambient temperature, an ambient humidity level, an ambient noise level, an ambient audio setting, etc. The in vehicle control system may determine additional settings, or further adjust the determined settings, of the one or more target vehicle systems based on the input received from the mobile device. Control instructions pertaining to the adjusted settings may then be delivered to the target vehicle systems or components.

At 312, the method 300 includes receiving input from the monitoring devices via the in vehicle computing system at a pre-determined time after vehicle operator initiates driving. Specifically, the input may be received at the in vehicle computing system from one or more of the plurality of monitoring devices either continuously or discontinuously based on the physiological parameter monitored by a specific monitoring device. Some of the physiological parameters may not be monitored continuously. In this case, the input relies on discontinuous input data. As described above, the monitoring devices may be worn by a vehicle operator and may include one or more sensors such as the heart rate sensor, the forehand temperature sensor, the blood pressure sensor, the blood glucose sensor, the nose area temperature sensor, the blood oxygenation sensor, the brain wave sensor, the perspiration level sensor, and the like. Other examples of monitoring device may include the camera that monitors muscular activity and pupil activity or pupil diameter of the vehicle operator.

Receiving input from the monitoring devices includes receiving sensor signals from the various wearable sensors based on interaction of the vehicle operator with the monitoring devices. As describe above, one example wherein the monitoring devices is communicatively coupled to the in vehicle computing system via a communication link or network, receiving input from the monitoring devices may include directly receiving input from the monitoring devices. Another example wherein the monitoring devices is communicatively coupled to a mobile device (e.g., via a communication link or network) and the mobile device, in turn, is communicatively to the in vehicle computing system via a communication link or network, receiving input from the monitoring devices includes indirectly receiving input from the monitoring devices via the mobile device.

At 314, the method includes processing the input to determine cognitive state via the in vehicle computing system. As described above, the input received from the monitoring devices may be indicative of various aspects of the physical state of the vehicle operator. Different changes to the various aspects of the physical state of the vehicle operator described above may allow the cognitive state of the vehicle operator to be determined. In particular, a stress level of the vehicle operator may be determined in response to changes in the various aspects of the physical state.

The input may be processed to determine the cognitive state of the vehicle operator after the vehicle is in operation. The cognitive state may be compared to the baseline of the cognitive state of the vehicle operator prior to the vehicle operator entering the vehicle. Specifically, the input may be processed and compared to the baseline cognitive state prior to entering the vehicle to determine whether the stress level of the vehicle operator after entering the vehicle increased or decreased. In particular, the in vehicle computing system may determine whether the input values correlate to physiological indicators of stress. The same physiological indicators monitored to determine the initial cognitive state may be utilized to determine the cognitive state of the vehicle operator the pre-determined time duration after the vehicle operator enters the vehicle and initiates driving.

At 316, the method 300 includes determining whether remediation measures may be implemented. As described herein, determining whether remediation measures may be implemented may be based on whether the input values correlate to physiological indicators of stress, historical information, location data, environmental factors, and how the initial cognitive state of the vehicle operator compares to the cognitive state of the vehicle operator after the vehicle operator enters the vehicle. Determining whether the input values correlate to physiological indicators of stress may be based on a threshold for the physiological indicators. Input values exceeding the threshold may correlate to higher stress levels of the vehicle driver. Each parameter set, as described above, in the plurality of parameters sets may have a corresponding threshold for each physiological indicator.

In one example, the initial input values of the monitoring devices may have correlated to physiological indicators of stress. However, after entering the vehicle and initiating driving, the input values may no longer be correlated to physiological indicators of stress. In this example, based on historical information and location data, a particular location may be correlated with physiological indicators of stress. Once the vehicle operator enters the care and initiates driving, the cognitive state of the vehicle operator may change without implementing remediation measures in response to the vehicle operator no longer being in contact with the stressor.

By comparing the baseline level of the cognitive state of the vehicle operator with a current cognitive state of the vehicle operator, the in vehicle computing system may more accurately determine whether implementing remediation measures will reduce the stress level of the vehicle operator due to vehicle stress, returning the stress level to a normal level. If it is determined physiological indicators of stress falsely indicate higher stress levels and implementing remediation measures may not reduce undesired stress levels of the vehicle operator, the method 300 may then end.

In contrast, the historical information and location data may not identify a location correlating to higher stress levels of the vehicle operator and/or locations correlating to physiological indicators of stress that falsely simulate higher stress levels of the vehicle operator. As such, the physiological indicators of stress may indicate higher stress levels of the vehicle operator. For example, the initial input values may not have correlated to physiological indicators of stress. However, after entering the vehicle and initiating driving, the vehicle operator may encounter heavy traffic and may experience an elevated vehicle cabin noise level due to an infant. Under the aforementioned conditions, the vehicle operator may experience higher levels of stress that may be alleviated by implementing remediation measures. If it is determined that physiological indicators of stress indicate higher stress levels and implementing remediation measures may reduce undesired stress levels of the vehicle operator, the method 300 includes determining remediation measures based on the initial cognitive state of the vehicle operator via in vehicle computing system at 318.

At 318, the method includes, determining remediation measures based on cognitive state via the in vehicle computing system. To establish a normal cognitive state of the vehicle operator, a plurality of vehicle settings may be adjusted to alleviate stressors. Specifically, one or more vehicle settings may be selected based on the input received from the monitoring devices. The one or more vehicle settings may be selected to decrease heart rate, increase high frequency components of heart rate variability, decrease blood pressure, decrease muscular activity, increase alpha waves, decrease pupil diameter, increase standard deviation of pupil diameter, decrease blinking to normalize blinking, decrease later gaze disengagement to normalize gaze disengagement, decrease peak saccade velocity to normalize peak saccade velocity, decrease forehand temperature, increase nose area temperature, and decrease temperature difference between forehand and nose.

Referring to the example above wherein the initial input values may not have correlated to physiological indicators of stress. Instead, after entering the vehicle and initiating driving, the vehicle operator may have encountered heavy traffic and experienced an elevated vehicle cabin noise level due to the infant. In the example, the vehicle operator may be experiencing physiological indicators of stress that may be reduced in response to remediation measures being implemented. In particular, the physiological indicators may be alleviated by adjusting the vehicle settings described herein.

For example, to relieve stress and alleviate the physiological indicators of stress, the in vehicle computing system (e.g., infotainment system) may adjust the vehicle settings. In one example, to reduce stress associated with the heavy traffic, the navigation system may suggest an alternate route to the desired destination with less traffic and unfavorable driving conditions in response to detecting physiological indicators of stress. As another example, to reduce the stress associated with the elevated vehicle cabin noise level due to the infant, the audio system may play soothing music to reduce the stress level of the vehicle operator due to vehicle stress in addition to the infant in response to detecting physiological indicators of stress.

The in vehicle computing system (e.g., infotainment system) may implement remediation measures continuously or discontinuously based on input received from the plurality of monitoring devices. Other embodiment so the present disclosure may utilize alternative or additional remediation measures to reduce the stress level of the vehicle operator due to vehicle stress than described herein (e.g., HMI/display content, scent, seat features, and the like). The remediation measures may be considered predictive, curative, or supportive.

At 310, the method includes transmitting remediation measures to corresponding systems via in vehicle computing system. The in vehicle computing system may implement the remediation measures to the appropriate vehicle systems to apply the selected settings to the target vehicle systems. For example, if the input from the monitoring devices is indicative elevated vehicle cabin noise levels, the in vehicle computing system may adjust the noise level in the vehicle cabin. Thus, the in vehicle computing system may automatically adjust the audio system settings to, for example, decrease sounds of audio being output by loudspeakers in the vehicle and/or playing soothing music. Accordingly, settings for the loudspeakers may be determined and transmitted to the audio system. Additionally, if the input from the monitoring devices is indicative of physiological indicators of stress due to traffic, the in vehicle computing system may adjust the route of the vehicle. In this way the in vehicle computing system may automatically adjust the navigation system setting to, for example, select a route with less traffic and more desirable driving conditions. As such, setting for the GPS in the vehicle may be determined and transmitted to the navigation system.

In this way, the in vehicle computing system automatically adjusts the vehicle settings by receiving sensed information from a monitoring devices and/or a mobile device and by using the sensed information before receiving some any sort of user command from the vehicle operator. This includes before receiving a command from the vehicle operator at the in vehicle computing system, such as via the vehicle operator pushing a button, or touch screen, etc., as well as before receiving a command from the vehicle operator at the in vehicle computing system via the monitoring devices or the mobile device, such as via the vehicle operator pressing a button of the mobile device, providing a voice command from the mobile device to the in vehicle computing system, and the like.

In an example, a method may be provided where an operator's estimated stress level is compared to a non-vehicle-cabin threshold outside the vehicle cabin and adjusting operation of the vehicle based on the comparison before the operator enters the cabin, and then additionally comparing the operator's estimated stress level to a vehicle-cabin threshold inside the vehicle cabin and adjusting operation of the vehicle based on the additional comparison once the operator enters the vehicle. For example, before entering the vehicle the operator may have a first level of stress that would indicate quiet music would be helpful to reduce stress upon entry into the cabin, however, after entry and determination that the operator intends to drive on a busy road, the operator's stress level may be lower than desired and so quiet music no longer be desired and alternative actions are taken to increase the operator's stress to the desired minimum level.

In an example, a method is provided for estimating a vehicle operator's stress responsive to various operating parameters, and adjusting a vehicle parameter in response thereto. The method may optionally include determining the operator's stress based on different parameters depending on the driving situation, and/or depending on whether the operator is in the vehicle or exterior to the vehicle. The method may optionally include comparing the operator's stress to both lower and upper thresholds and adjusting vehicle parameters to maintain the stress level between the lower and upper thresholds. The method may optionally include varying the upper and/or lower thresholds depending on whether the operator is operating the vehicle or parked, and/or whether the operator is inside the cabin or exterior to the cabin.

The technical effect of determining a cognitive state of a vehicle operator associated with physiological indicators of stress and implementing remediation measures in response to detecting physiological indicators of stress include increasing driver satisfaction while reducing unfavorable driving conditions for the vehicle operator.

As used herein, an element or step recited in the singular and proceeded with the word "a" or "an" should be understood as not excluding plural of said elements or steps, unless such exclusion is stated.

Furthermore, references to "one embodiment" or "one example" of the present disclosure are not intended to be interpreted as excluding the existence of additional embodiments that also incorporate the recited features.

As used herein, terminology in which "an embodiment," "some embodiments," or "various embodiments" are referenced signify that the associated features, structures, or characteristics being described are in at least some embodiments, but are not necessarily in all embodiments. Moreover, the various appearances of such terminology do not necessarily all refer to the same embodiments, and are not intended to be interpreted as excluding the existence of additional embodiments that also incorporate the recited features.

As used herein, terminology in which elements are presented in a list using "and/or" language means any combination of the listed elements. For example, "A, B, and/or C" may mean any of the following: A alone; B alone; C alone; A and B; A and C; B and C; or A, B, and C.

As used herein, the term "substantially similar to" is construed to mean the same as with a tolerance for variation that a person of ordinary skill in the art would recognize as being reasonable.

As used herein, terms such as "first," "second," "third," and so on are used merely as labels, and are not intended to impose numerical requirements or a particular positional order on their objects.

## Claims

1. A method of relieving stress for an operator of a vehicle, comprising:
receiving a set of inputs from a plurality of monitoring devices after the operator begins operation of the vehicle;
processing the set of inputs to determine a stress level of the operator of the vehicle;
comparing the stress level to a pre-determined stress level threshold;
implementing a first remediation measure based at least upon the stress level not being within the pre-determined stress level threshold and being lower than the pre-determined stress level threshold;
implementing a second remediation measure based at least upon the stress level not being within the pre-determined stress level threshold and being lower than the pre-determined stress level threshold,
wherein the second remediation measure is different from the first remediation measure.

2. The method of relieving stress for the operator of the vehicle of claim 1, wherein the set of inputs is a second set of inputs, the plurality of monitoring devices is a second plurality of monitoring devices, and the stress level is a second stress level, comprising:
receiving a first set of inputs from a first plurality of monitoring devices before the operator of the vehicle begins operating the vehicle; and
processing the first set of inputs to determine a first stress level of the operator of the vehicle,
wherein at least one of the implementation of the first remediation measure and the implementation of the second remediation measure is further based upon the second stress level being higher than the first stress level.

3. The method of relieving stress for the operator of the vehicle of claim 2,
wherein the first plurality of monitoring devices is different than the second plurality of monitoring devices.

4. The method of relieving stress for the operator of the vehicle of any of claims 2 through 3,
wherein each of the first set of inputs and the second set of inputs includes a plurality of physiological parameters selected from outputs of: a heart rate sensor, a forehand temperature sensor, a blood pressure, a blood glucose sensor, a blood oxygenation sensor, a brain wave sensor, a perspiration level sensor, a camera for monitoring muscular activity, and a camera for monitoring pupil activity or pupil diameter.

5. The method of relieving stress for the operator of the vehicle any of claims 2 through 4,
wherein a mobile device of the operator includes the first plurality of monitoring devices

6. The method of relieving stress for the operator of the vehicle of claim 5,
wherein each of the first set of inputs is received from the mobile device of the operator via a wireless communication interface of the vehicle.

7. The method of relieving stress for the operator of the vehicle of any of claims 1 through 6,
wherein each of the first remediation measure and the second remediation measure is implemented by at least one of: an infotainment system, a navigation system, an Advance Driver Assistance System (ADAS), and a Voice Personal Assistant (VPA); and/or
wherein the set of inputs corresponds with a set of physiological indicators of stress.

8. A method of relieving stress for an operator of a vehicle, comprising:
receiving a first set of input values from a first plurality of monitoring devices before the operator begins operation of the vehicle;
processing the first set of input values to establish a baseline cognitive state of the operator;
receiving a second set of input values from a second plurality of monitoring devices after the operator begins operation of the vehicle;
processing the second set of input values to establish a current cognitive state of the operator;
determining a remediation measure to reduce a stress level of the operator based at least in part upon a comparison of the current cognitive state to the baseline cognitive state; and
implementing the remediation measure.

9. The method of relieving stress for the operator of the vehicle of claim 8,
wherein a set of physiological indicators of stress is correlated with the second set of inputs; and
wherein the determination of the remediation measure to reduce the stress level of the operator is based additionally upon whether the set of physiological indicators exceeds a stress level threshold for the set of physiological indicators.

10. The method of relieving stress for the operator of the vehicle of any of claims 8 through 9,
wherein the stress level threshold for the set of physiological indicators is based upon at least one of the first set of input values and the baseline cognitive state.

11. The method of relieving stress for the operator of the vehicle of any of claims 8 through 10,
wherein the first plurality of monitoring devices is different than the second plurality of monitoring devices.

12. The method of relieving stress for the operator of the vehicle of any of claims 8 through 11,
wherein one or more physiological indicators of stress is correlated with the second set of inputs; and
wherein the remediation measure includes one or more vehicle settings.

13. The method of relieving stress for the operator of the vehicle of claim 12,
wherein, when the one or more physiological indicators of stress include an increased heart rate, the determined remediation measure is to decrease heart rate;
wherein, when the one or more physiological indicators of stress include reduced high-frequency components of heart rate variability, the determined remediation measure is to increase high-frequency components of heart rate variability;
wherein, when the one or more physiological indicators of stress include an increased blood pressure, the determined remediation measure is to decrease blood pressure;
wherein, when the one or more physiological indicators of stress include suppressed alpha waves of the brain, the determined remediation measure is to increase alpha waves of the brain;
wherein, when the one or more physiological indicators of stress include an increased forehand temperature, the determined remediation measure is to decrease forehand temperature;
wherein, when the one or more physiological indicators of stress include a decreased nose area temperature, the determined remediation measure is to increase nose area temperature;
wherein, when the one or more physiological indicators of stress include an increase in difference between forehand temperature and nose area temperature, the determined remediation measure is to decrease differences between forehand temperature and nose area temperature;
wherein, when the one or more physiological indicators of stress include an increased muscular activity, the determined remediation measure is to decrease muscular activity;
wherein, when the one or more physiological indicators of stress include an increased pupil diameter, the determined remediation measure is to decrease pupil diameter;
wherein, when the one or more physiological indicators of stress include a decreased standard deviation of pupil diameter, the determined remediation measure is to increase standard deviation of pupil diameter;
wherein, when the one or more physiological indicators of stress include an increased blinking, the determined remediation measure is to decrease blinking;
wherein, when the one or more physiological indicators of stress include a later gaze disengagement, the determined remediation measure is to decrease later gaze disengagement; and
wherein, when the one or more physiological indicators of stress include an increased peak saccade velocity, the determined remediation measure is to decrease peak saccade velocity.

14. A system for relieving stress for an operator of a vehicle, comprising:
a processor;
and a memory storing instructions that, when executed, cause the processor to:
receive a first set of inputs from a first plurality of monitoring devices before the operator begins operation of the vehicle;
process the first set of inputs to establish a baseline cognitive state of the operator;
receive a second set of inputs from a second plurality of monitoring devices after the operator begins operation of the vehicle;
process the second set of inputs to establish a current cognitive state of the operator; and
determine whether the second set of inputs correlates to one or more physiological indicators of stress;
determine whether a remediation measure will reduce a stress level of the operator based at least in part upon a comparison of the current cognitive state to the baseline cognitive state;
implement the remediation measure when the second set of inputs correlates to one or more physiological indicators of stress and the remediation measure will reduce the stress level of the operator; and
refrain from implementing the remediation measure when the second set of inputs does not correlate to the one or more physiological indicators of stress or the remediation measure will not reduce the stress level of the operator.

15. The system for relieving stress for the operator of the vehicle of claim 14,
wherein each of the first set of inputs and the second set of inputs includes a plurality of physiological parameters selected from outputs of: a heart rate sensor, a forehand temperature sensor, a blood pressure, a blood glucose sensor, a blood oxygenation sensor, a brain wave sensor, a perspiration level sensor, a camera for monitoring muscular activity, and a camera for monitoring pupil activity or pupil diameter;
wherein each of the remediation measure is implemented by at least one of: an infotainment system, a navigation system, an Advance Driver Assistance System (ADAS), and a Voice Personal Assistant (VPA);
wherein the remediation measure includes one or more vehicle settings;
wherein the first plurality of monitoring devices is different than the second plurality of monitoring devices;
wherein a mobile device of the operator includes the first plurality of monitoring devices; and/or
wherein each of the first set of inputs is received from the mobile device of the operator via a wireless communication interface of the vehicle.
